# EUROPEAN PATENT APPLICATION

(11) **EP 2 022 420 A2**
(43) Date of publication of application: **11.02.2009**
(21) Application number: 08252584.1
(22) Date of filing: 30.07.2008
(51) Int. Cl.: A61B 17/32

(54) **Shaped motorized skin-treatment device**

(30) Priority: 31.07.2007 US 953065 P
(71) Applicant: Johnson and Johnson Consumer Companies, Inc., Skillman, NJ 08558 (US)
(72) Inventor: Gubernick, David, Cherry Hill, NJ 08003 (US); Hull, Raymond J., Jr., Hampton, NJ 08827 (US); Menke, James, Califon, NJ 07830 (US); Rytel, John F., East Brunswick, NJ 08816 (US)
(74) Representative: Kirsch, Susan Edith

(57) **Abstract**

A handheld, motorized apparatus (3) is arranged and configured to impart motion to skin placed in contact therewith. The motorized apparatus includes a substantially planar attachment surface (19a,b) suitable for coupling a skin-contactable element (9) thereto; a motor (21) within said body; and means for transferring mechanical energy from the motor to the skin-contactable element in order to impart motion to the skin contactable surface (9). The apparatus (3) has a maximum linear dimension of less than about 13 cm.

## Description

### BACKGROUND OF THE INVENTION

Numerous techniques have been proposed to provide cosmetic and/or or skin cleansing or rejuvenation benefits. Examples of such techniques include surfactant-based cleansing formulations, suspensions of abrasives, and/or wipes that are manually massaged against the skin. As an alternative to manual application of such treatments, a motorized skin-treatment device may be employed, such as in conjunction with a cleanser in order to provide such benefits.

Rhoades, US Pat. No. 6,652,888, purports to disclose a method for skin rejuvenation with buffing cream. In this method, a cream moisturizer for resurfacing human skin is provided with a suspension of microcrystals of alumina in a ratio of approximately 14 grams per ounce of moisturizer cream. The cream moisturizer is buffed into the epidermal layer of the skin with a closed-cell sponge pad driven by a vibrator. The alumina microcrystals buffs an epidermal layer off the skin to provide a soft smooth surface, thereby rejuvenating the skin.

Dotan, US Pat. No. 6,139,553, purports to disclose a facial treatment implement and method. In this disclosure, an implement for and a method of, facial treatment are described in which a facial preparation containing an abrasive is applied to the face, and a facial treatment head is pressed against the facial preparation and is vibrated to work the facial preparation into the skin of the subject's face. Preferably, the facial treatment head includes a convexly-curved surface, but may also be one including a rotary disk, which is rotated and vibrated while pressed into contact with the facial preparation on the subject's face. The facial preparation is preferably a mud mixture; preferably, this treatment is followed by another one utilizing a facial preparation containing a moisturizer, which is also worked into the skin in a similar manner as the mud treatment.

Zelickson, US Pat. No. 6,645,184 purports to disclose a tape stripping system and method. The system uses a tape-like material by applying it to human skin and then stripping the tape from the skin to remove excess or unwanted material. The system further includes application of other elements to facilitate skin recovery and rejuvenation processes.

We have recognized that while motorized, skin-treatment devices are commercially available, these systems, while efficacious, may be less than optimal for various reasons. Concerns that smaller devices are less effective have kept these devices rather large. However, we have also recognized that available motorized, skin-treatment devices are often somewhat heavy and/or bulky and therefore difficult to maneuver, obscure one's ability to see the skin surface being treated and have unnecessarily high packaging costs. We have further recognized the need to overcome one or more of the above-mentioned drawbacks, yet still be able to maintain the efficacy of skin treatment.

### SUMMARY OF THE INVENTION

In one aspect, embodiments of the invention relate to a handheld, motorized apparatus arranged and configured to impart motion to skin placed in contact therewith, wherein the motorized apparatus includes an attachment surface suitable for coupling a skin contactable element thereto; a motor within said body; and a means for transferring mechanical energy from the motor to the skin-contactable element in order to impart motion to the skin contactable surface; wherein said apparatus has a maximum linear dimension of less than about 13 cm.

In another aspect, embodiments of the invention relate to a handheld, motorized apparatus arranged and configured to impart motion to skin placed in contact therewith, wherein the motorized apparatus includes a body. The body includes an attachment surface suitable for coupling a skin contactable element thereto; a first gripping surface; a second gripping surface opposed to said first gripping surface, wherein said first gripping surface and said second gripping surface are suitable for gripping between a thumb and fingers of a user's hand, and wherein when said device being held in said manner does not necessarily contact a palm of said user. The apparatus further includes a motor within said body and a means for transferring mechanical energy from the motor to the skin-contactable element in order to impart motion to the skin contactable surface. The apparatus has a maximum linear dimension of less than about 13 cm.

In another aspect, embodiments of the invention relate to a handheld, motorized apparatus arranged and configured to impart motion to skin placed in contact therewith, wherein the motorized apparatus comprises a substantially planar attachment surface suitable for coupling a skin-contactable element thereto; a motor within said body; and means for transferring mechanical energy from the motor to the skin-contactable element in order to impart motion to the skin contacable surface; wherein said apparatus has projected area less than about 100 cm², wherein said projected area is the area projected in a direction normal to said substantially planar surface, and wherein said apparatus has a dimension that is measured from said substantially planar attachment surface along said direction to a point on said device that is most remote from said substantially planar attachment surface, and wherein said dimension is less than about 11 cm.

In another aspect, embodiments of the invention relate to a kit that includes the apparatus described above and a skin-contactable element such as a skin-treatment pad.

In another aspect of the invention, a method of treating the skin of a subject includes energizing the motor of the apparatus described above, and contacting the skin with a skin-contactable element.

### BRIEF DESCRIPTION OF THE DRAWINGS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.
Figure 1 is a schematic side view of a system for treating the skin that is consistent with embodiments of the invention described herein;
Figure 2 is a front view of the system of Figure 1;
Figure 3 is the front view of the system of Figure 1, additionally depicting a user holding the apparatus consistent with embodiments of the invention;
Figure 4 is a top perspective view of the system of Figure 1;
Figure 5 is a bottom view of the system of Figure 1;
Figure 6 is a top view of a carrier and a skin-contactable element that may be used with the system of Figure 1;
Figure 7 is a cross sectional view of Figure 1, taken through line 1-1' of Figure 1; and
Figure 8 is a perspective view of a motor suitable for use in the system of Figure 1.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

As used herein the specification and the claims, the term "mechanical skin resurfacing technique" and variants thereof relate to the mechanically assisted removal of mammalian (especially human) skin cells, ranging from mild techniques (such as exfoliation and abrasive cleansing) through microdermabrasion, and up to severe techniques such as dermal abrasion.

As used herein the specification and the claims, the term "dermabrasion" and variants thereof relate to a non-thermal resurfacing technique especially well suited for deep defects of the skin such as acne scars, heavy wrinkles and the disfiguring effects of skin conditions like rosacea. The procedure involves the mechanical sanding of the upper layers of the skin and penetrates the skin deeper than microdermabrasion. With dermabrasion, a new layer of skin replaces the abraded skin during healing, resulting in a smoother appearance

As used herein the specification and the claims, the term "microdermabrasion" and variants thereof relate to a very mild and less-penetrating form of dermabrasion, more suited for reduction of fine lines and wrinkles and for other less severe skin conditions. Microdermabrasion penetrates less deeply into the skin, primarily the stratum corneum, or portions thereof.

As used herein the specification and the claims, the term "exfoliation" and variants thereof relate to the peeling and sloughing off of the skin's tissue cells.

As used herein the specification and the claims, the term "cleansing" and variants thereof relate to removal of dirt, oils, make-up and the like from the surface of the skin, especially through surfactant washing, and perhaps also penetrating into the pores of the skin. In "abrasive cleansing," some degree of exfoliation may also occur.

These mechanical skin treatments may facilitate the delivery of benefit agents to skin tissue, e.g., cleansing and the delivery of acne treatment compositions or rejuvenating agents such as retinol.

As used herein the specification and the claims, the term "nonwoven" and variants thereof relate to a sheet, web, or batt of natural and/or man-made fibers or filaments, excluding paper, that have not been converted into yarns, and that are bonded to each other by any of several means. For additional clarification, nonwovens are distinct from woven and knitted fabrics. The fibers included in the nonwoven materials may be staple or continuous or be formed in situ, and preferably, at least about 50% of the fibrous mass is provided by fibers having a length to diameter ratio greater than about 300:1.

The present invention is directed to systems, articles, compositions, and methods useful for mechanical skin resurfacing techniques employing a handheld motorized device. In various embodiments of the invention, such systems, articles, and methods provide a unique combination of high reliability and convenience for the user, as well as a highly efficacious mechanical skin resurfacing technique.

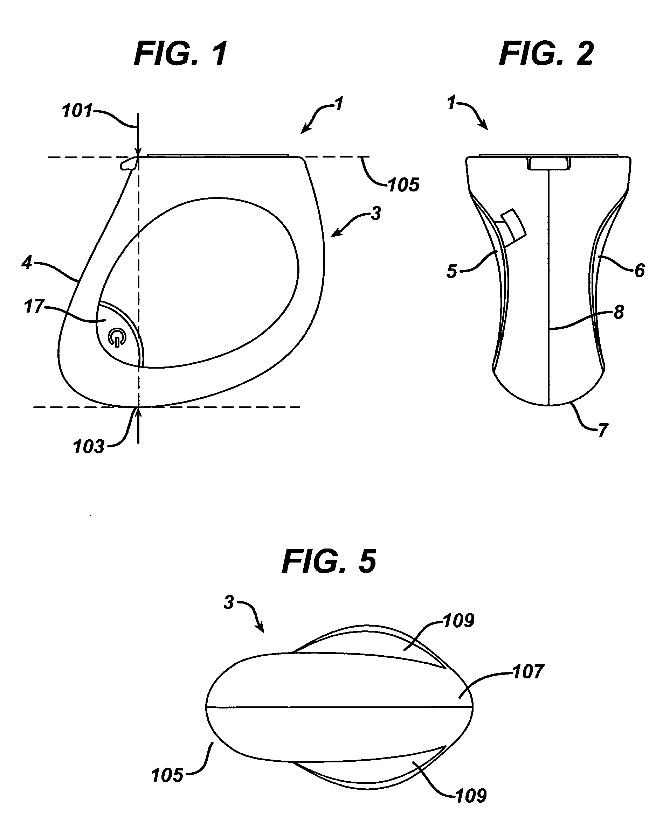

Figure 1 depicts a side view of one non-limiting example of a system 1 useful for mechanical skin resurfacing according to embodiments of invention described herein. Figure 2 depicts a front view of the system of Figure 1.

The system 1 includes a motorized apparatus 3 that is generally shaped to be held in a hand of a user. The apparatus 3 may be of varying shapes and dimensions. In one notable embodiment, the apparatus 3 includes a first gripping surface 5. First gripping surface 5 may terminate in a basal surface 7. The apparatus may further include a second gripping surface 6 that is opposed to the first gripping surface 5. Gripping surfaces 5, 6 are preferably convex. By "convex" it is meant that the gripping surface 5 bulges out away from a plane of symmetry 8 of the apparatus 3. In one embodiment, the first gripping surface 5 and the second gripping surface 6 have a minimum separation 20 that is from about 10cm to about 50 cm, preferably from about 15 cm to about 30cm. By "minimum separation" it is meant the smallest linear distance that separates the gripping surfaces 5, 6.

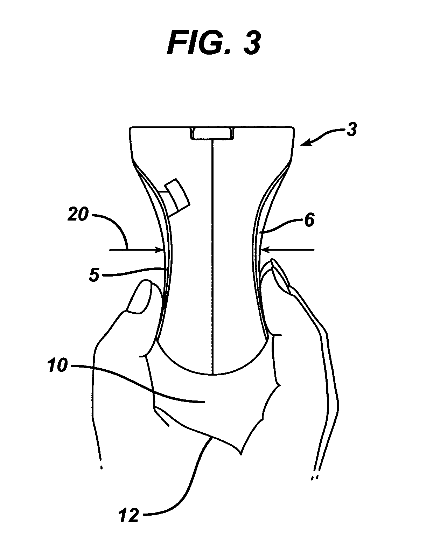

In one embodiment, the first gripping surface 5 and the second gripping surface 6 are suitable for gripping between a thumb and fingers of a user's hand. The first gripping surface 5 and the second gripping surface 6 are preferably configured such that apparatus 3 when held in this manner does not necessarily contact the palm of the user's hand. This so-called "precision grip" is shown in Figure 3. As shown in the figure, the apparatus 3 can be comfortably gripped such that the thumb is on the first gripping surface 5 and one or more fingers are on the second gripping surface 6 such that the users palm is not on the apparatus 3 and a space 10 exists between the thumb or fingers and the hand "proper" 12.

In order to facilitate the ability of the user to hold the apparatus 3 as above in the precision grip, in one preferred embodiment, the apparatus 3 has a maximum linear dimension that is less than about 13 centimeters. By "maximum linear dimension" it is meant that length of the longest line that could be drawn from one portion of the apparatus 3 to another portion of the apparatus 3. To provide even a higher level of compacted-ness, the maximum linear dimension of the apparatus 3 may be less than about 11 cm, more preferably less than about 10 cm, even more preferably less than about 9 cm.

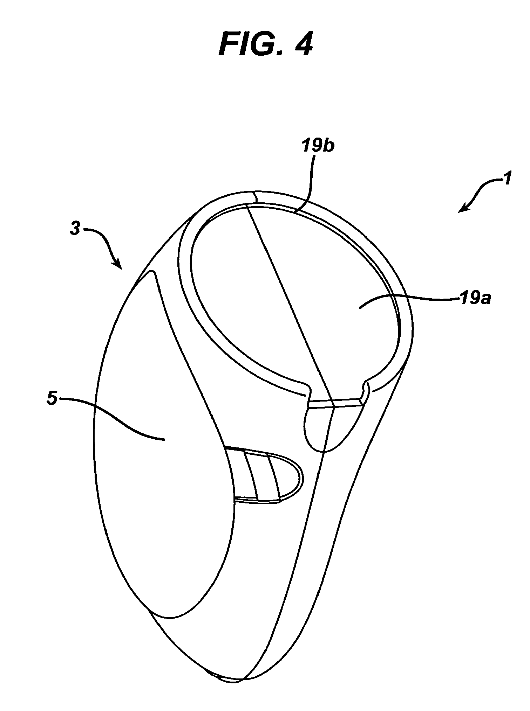

Figure 4 depicts a top perspective view of system 1. The apparatus 3 may include one or more attachment surfaces 19a,b for removably attaching a skin-contactable element thereto. The term, "removably attaching," and variants thereof, relate to the ability to attach, remove, and reattach the element without significantly compromising the attachment strength. In one embodiment, the attachment surface has an area of at least about 5cm², preferably at least about 10 cm².

Figure 4 depicts one preferred embodiment in which attachment surface 19a is substantially planar. By substantially planar, it is meant a surface that over its entire extent (e.g., an area of at least about 5cm², preferably at least about 10cm²) has minimal to no curvature (e.g., has a radius of curvature of at least about twice times the maximum linear dimension of the attachment surface 19a, preferably at least about 5 times the maximum linear dimension of the attachment surface 19a). The substantially planar surface has maximum length and width dimensions substantially greater than its maximum depth dimension. For example, a 5 to 10 cm², would have a maximum depth dimension of about 1 mm or less. A substantially planar attachment surface facilitates the attachment of a substantially planar skin-contactable element thereto, providing ease of skin treatment

In another embodiment, the apparatus has a combination of attributes in that it has both (1) a limited dimension as measured normal to the substantially planar attachment surface and (2) a limited projected area in a plane normal to the substantially planar attachment surface. Referring again to Figure 1, in this embodiment, the apparatus has a dimension 101 that is measured from substantially planar attachment surface 19a normal to a point 103 on the apparatus 3 that most remote from a plane 105 defined by the substantially planar attachment surface 19a. The dimension 101 is desirably less than about less than about 11 cm, more preferably less than about 10 cm, even more preferably less than about 8 cm.

Figure 5 depicts a bottom plan view of the apparatus 3, a view of the apparatus 3 that is perpendicular to plane 105 of Figure 1. In one embodiment, the apparatus 3 has a projected area 105 (the area projected onto a plane parallel to substantially planar attachment surface 19a. - i.e., projected in a direction normal to substantially planar attachment surface 19a) that is less than about 100 cm², preferably less than about 80 cm², more preferably less than about 60 cm², even more preferably less than about 50 cm², and most preferably less than about 30 cm². Note that projected area 105 includes the entire cross-hatched area in Figure 5, i.e., a first projected area 107 associated with a lower portion if the apparatus 3, and second projected areas 109 that are associated with a top portion of the device. The first projected area 107 is separated from the second projected areas 109 by a portion of the device that is defined by inwardly sloping portions of the convex surfaces 5, 6 noted in Figure 2.

While Figures 1-5 depict a particular shape of the apparatus 3, other shapes are also suitable. For example, the apparatus 3 may have an hourglass shape (e.g., a cylindrical shape that is "pinched" in the center), a disk or puck shape (e.g., a disk with indentations for finger gripping), among other shapes that may or may not facilitate a precision grip. The apparatus 3 may include other components useful for attaching the device to a hand of a user - e.g., the apparatus 3 may include one or more straps, bands, or pockets that hold the apparatus 3 against the hand, fingers, thumb, or combinations thereof.

In one embodiment, the apparatus 3 defines a surrounds a volume that is less than about 130 cm³, preferably less than about 100 cm³. The mass of the apparatus 3 (including any batteries) may be less than about 125 grams, preferably less than about 100 grams.

The inventors have found that by utilizing one or more of the above described design features, the apparatus 3 generally provides one or more highly desirable features: fits within a user's palm, can be held in a precision grip, has enhanced ease of manipulation of the apparatus 3, and is not excessively heavy. Furthermore, without wishing to be bound by theory, it is believed that by making the apparatus 3 relatively small and shaped as described, and facilitating the apparatus 3 to be held in the precision grip, it is believed that the user can reduce the amount of vibration that is dissipated into the hand, thereby reducing discomfort with the device and enhancing the vibration that is directed into the skin to be treated.

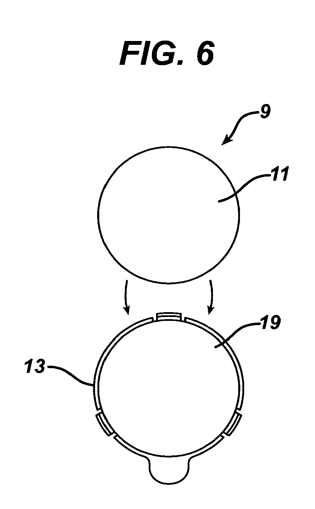

Figure 6 depicts a top view of a skin-contactable element 9 (e.g., a free-standing mass such as a sponge, a fibrous material or other material, or combinations thereof, including those described in this specification, below) that includes a skin-contactable surface 11 for contacting the skin. The skin-contactable element 9 may be placed (as indicated by the arrows in Figure 6) onto and secured to an optional carrier 13 (e.g., a firm plastic substrate). The skin-contactable element 9 may be secured to the carrier by any of various means, e.g., microhooks, adhesive, and the like. The carrier 13 may be removably attached and detached from the one or more surfaces 19 of the apparatus 3 via snap, threaded screw, friction fit or otherwise

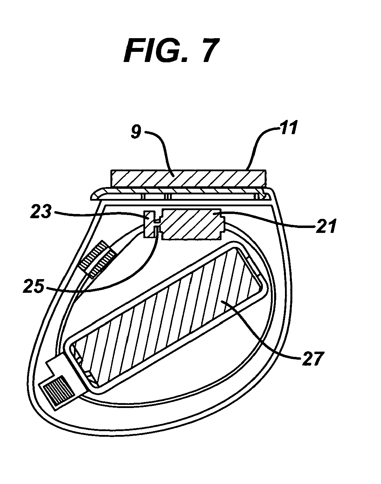

Figure 7 depicts a cross-sectional view taken through line 1-1' shown in phantom in Figure 1. A user grasping the gripping surfaces 5, 6 activates a motor 21 within the apparatus 3, such as by actuating a switch 17 (shown in Figure 1) on the apparatus 3, allowing stored energy from a battery 27 to energize the motor 21. The battery 27 may be housed in a chamber that is accessed via a snap 18 (shown in Figure 4).

The motor 21, thereby energized, provides mechanical energy that is transmitted to the attached skin-contactable surface 11 and to an expanse of skin (not shown) placed in contact therewith. The mechanical energy may comprise a vibrational form that is transmitted via an eccentric weight 23 on a rotating shaft 25.

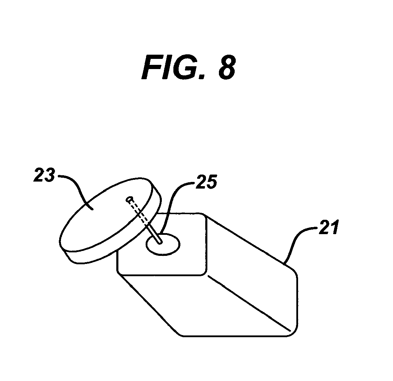

In order facilitate the transmission of energy from the motor to the skin-contactable element 9, the motor 21 may be positioned in close proximity to the skin-contactable element 9 (Figure 8 shows a perspective view of a suitable motor). While the axis of the shaft 25 is desirably parallel to the skin-contactable surface 11, as shown in Figure 6, this is not required.

Applicants have surprisingly found that the energy provided by the apparatus 3 need not be high in order to provide skin benefits. In particular, Applicants have surprisingly found for embodiments in which the energy is provided by vibration, that a small motor, such as one that may be used for communicating an incoming call or message from a cellular phone, a pager, or a personal digital assistant may be suitable. The motor utilizes an eccentric weight having a mass of less than 3 grams to provide a high level of cleansing and makeup removal. In one embodiment, the mass of the eccentric weight is less than about 2 grams. In another embodiment, the mass of the eccentric weight is from about 1.6 grams to about 3 grams.

The inventors have found that apparatuses of the present invention, not only are capable of providing a high level of cleansing and other skin benefits, but one can adjust the design of the apparatus to achieve additional benefits related to ease of use, and convenience. Specifically by reducing the mass of the eccentric weight 23, (1) one can reduce the space occupied by the motor and reduce the space occupied by the entire apparatus, making it easier to see areas of the skin when the apparatus is in use; (2) the apparatus can be made easier to transport; (3) the apparatus can be made such that is has reduced packaging costs; and (4) one can also reduce the weight of the apparatus 3 making it lighter and less tedious to hold for extended use periods.

According to embodiments of the invention, the apparatus 3 is placed in contact with the skin to be treated. While the above Figures depict contact with the skin via the skin-contactable element 9, this is not required. Contact with the skin may be for example via a plurality of skin-contactable surfaces from loose abrasive particles, e.g., mineral particles such as those including oxides of aluminum, mica, silicates and the like, as well as organic abrasive particles such as ground walnut shells, ground apricot shells, ground inorganic particles.

In order to avoid difficulties in removing/rinsing loose abrasive particles after use, in a preferred embodiment, contact is made with skin via a skin-contactable element, such as the skin-contactable element 9 described in relation to the above-mentioned Figures. The skin-contactable element is generally a pad suitable for contacting or treating the skin, such as a freestanding mass such as a sponge, a fibrous material or other material, or combinations thereof. The skin-contactable element may have little to no inherent abrasiveness, or may have inherent abrasiveness or, alternatively has an abrasive system bound thereto to provide abrasiveness.

The skin-contactable element 9 may comprise, consists essentially of, or consists of a fibrous material. Suitable fibrous materials include, without limitation, woven, nonwoven (oriented, e.g., via a carding process, or non-oriented), or knit fabrics. The fibers may be integrated into a nonwoven structure via, for example, needle punching, through-air bonding, hydro entangling, spun-bonding, chemical bonding (including adhesive bonding), or mechanical processing (such as embossing).

The fibers may thereby be arranged into a freestanding fabric (e.g., a porous fabric). The nonwoven fabric may have an average pore diameter (as calculated via Cohen, "A Wet Pore-Size Model for Coverstock Fabrics," Book of Papers: The International Nonwovens Fabrics Industry, pp.317-330, 1990) that is from about 150 microns to about 500 microns, such as from about 220 microns to about 400 microns. A representative, non-limiting list of useful fibers includes fibers derived from organic polymers such as, for example, polyester, polyolefin, polyamide and rayon fibers and bicomponent fibers; cellulose-based fibers such as wood pulp, rayon, and cotton; and combinations thereof.

In order to provide a particularly suitable degree of cleansing or abrasion, the skin-contactable element 9 may include fibers are bonded via mechanical means such as a needle-punching process, known to those skilled in the art, such as to a thickness of about 0.5mm to about 5mm, more preferably from about 1mm to about 5mm. The fibrous material may have a basis weight (mass per unit area) sufficient to maintain its mechanical integrity for one or more uses of the skin-contactable element 29. The basis weight may be, for example, between about 10 grams per square meter (gsm) and about 450 gsm, such as between about 200 gsm and about 400 gsm, preferably between about 300 and about 400 gsm. The fibrous material desirably includes rayon to provide softness and a strong, resilient material such as an olefin or polyester. One particularly notable fibrous material is a needle-punched blend of staple-length 1.5 denier "TENCEL" rayon and staple-length 4-5 denier PET available from Precision Custom Coating of Totowa, NJ, with a basis weight of about 200 gsm and about 400 gsm.

The bound abrasive may be an abrasive system that is bound to fibers. The term an abrasive system "bound to fibers" refers to abrasive units, particles, aggregates, and the like that are firmly attached to the fibers and do not readily separate in use therefrom. Such abrasive may be bound by various means; one notable means is by chemical bonding (including, without limitation, adhesive bonding).

The abrasive system may include or consist essentially of a water insoluble abrasive material. In one embodiment of the invention, the abrasive system includes a resin or polymer. For example, the polymer may be a homopolymer, copolymer, or terpolymer, and may be a blend of two or more different polymers. The polymers may be random, block, star, or other known architecture. The polymer may be made by known means, such as emulsion polymerization, dispersion, suspension, or solution polymerization. In a preferred embodiment the polymer is formed by emulsion polymerization. The polymers may be non-functional, or may contain functionality designed to optimize the properties of the coating in the specific application. One of skill in the art will be able to adjust monomer content and architecture to improve end-use performance of the polymer composition. The polymer could be a synthetic polymer, or could be a natural polymer such as, for example, a polysaccharide, starch, modified starch, or guar gum. Preferred polymers include homopolymers and copolymers having one or more of the following monomers: (meth)acrylates, maleates, (meth)acrylamides, vinyl esters, itaconates, styrenics, unsaturated hydrocarbons and acrylonitrile, nitrogen functional monomers, vinyl esters, alcohol functional monomers. Particularly preferred monomers include, but are not limited to, vinyl acetate; methyl (meth)acrylate, ethyl (meth)acrylate, butyl (meth)acrylate, ethylene, vinyl chloride, and styrene.

If included in the skin-contactable element,, the polymer is selected so as to provide enough hardness so as to be abrasive to skin, but not so hard as to cause scratching or discomfort. In one embodiment of the invention, the polymer has a glass transition temperature, Tg greater than about -20 degrees Celsius (° C.), such as from about 0° C. to about 105° C. In one notable embodiment, the polymer has a T_{g} from about 0° C and about 50° C.

T_{g} can be determined by differential scanning calorimetry (DSC) conducted at a heating rate of 20.0 ° C./minute with 5 mg or smaller samples. The Tg is calculated as the midpoint between the onset and end of heat flow change corresponding to the glass transition on the DSC heat capacity heating curve. The use of DSC to determine T_{g} is well known in the art, and is described by B. Cassel and M. P. DiVito in "Use of DSC To Obtain Accurate Thermodynamic and Kinetic Data", American Laboratory, January 1994, pp 14-19, and by B. Wunderlich in Thermal Analysis. Academic Press, Inc., 1990.

The polymer may be a thermosetting polymer, (e.g., a polymer having crosslinks that are generally not reversible with changes in temperature). One notable polymer has an acrylic base/ vinyl acrylic base that is partially cross-linked during cure with a Tg of about 30° C., e.g., VINAMUL ABX 30, resin commercially available as from Celanese Corporation of Dallas, TX.

The abrasive system may further include one or more additional functional components compounded with the abrasive. Useful additional functional components include, but are not limited to plasticizers; cross-linkers; starch; polyvinyl alcohol; formaldehyde thermosetting agents such as melamine, urea, phenol; fillers; humectants; surfactants; salts; fragrances; and pigments or reflective agents. The additional functional components may be present in the abrasive system at from 0 to 20 percent by weight, and preferably from 5 to 15 percent by weight, calculated as a percent of the polymer solids.

The skin-contactable element 9 may be formed by depositing the abrasive system onto the fibers by various means known to the art of industrial polymer coating, such as slot coating, foam coating, saturation, printing, or spraying. Spraying is particularly notable to facilitate the formation of discrete abrasive units on top of the fibers so that waste is reduced and efficacy is optimized.

If the abrasive system is applied by spraying, a sprayable composition that includes the abrasive system (e.g., polymer plus other functional ingredients as well as water or another suitable carrier) may be sprayed onto the fibers followed by drying the resulting fiber/abrasive composite in a conventional oven. Although, the foregoing relates to a skin-contactable element that incorporates an abrasive system bound to the fibers, in one embodiment of the invention, the fibers themselves may be abrasive, without the need of including an additional abrasive system bound to the fibers. For example, in one particular embodiment, the skin-contactable element includes staple fibers that are integrated into a nonwoven structure via needle punching, through air bonding, or thermal bonding. The fibers may be high denier fibers formed from polyester; polyolefins; rayon fibers; bicomponent fibers; cellulose-based fibers such as wood pulp, rayon, and cotton; or combinations thereof.

The inventors have surprisingly found that one desirable attributes may be achieved by using moderately abrasive skin-contactable elements that have an appropriate "Durable Abrasiveness" determined according to the "Durable Abrasiveness Test" described in the "Test Methods" section, below. Furthermore, the above-mentioned benefits are further enhanced if the skin-contactable elements are selected based upon their Durable Abrasiveness in combination with one or more properties relating to how the pads behave under compressive load, specifically Compressibility and Displacement. These properties relate to the ability of the skin-contactable element to transfer the mechanical energy from the apparatus 3 to the skin-contacting surface in a moist or wet environment to mechanically resurface the skin.

In one embodiment, the skin-contactable element has a Durable Abrasiveness has a Durable Abrasiveness less than 14, such as from 2 to 14, preferably from about 2.5 to about 12, more preferably from about 3 to about 10, even more preferably from about 4 to about 9.

In certain embodiments, the inventors have also surprisingly found that moderately abrasive skin-contactable elements - particularly those meeting the fixed abrasiveness criteria as specified above, have enhanced performance when used in conjunction with a mechanical tool, when the skin-contactable element also has additional properties relating to their behavior under applied compressive load. In particular, the skin-contactable element provide some displacement under an applied load, but do not displace overly so.

Displacement of generally recoverable deformation due to an applied compressive force and Compressibility are additional properties useful to characterize the skin-contactable elements. These properties may be measured according to the "Compressibility and Displacement Test" described in the "Test Methods" section, below.

As such, in one embodiment, the skin-contactable element has a Displacement from 0.15 mm to about 2.0mm, preferably from about 0.25mm to about 1mm, more preferably from about 0.25 to about 0.8mm, and most preferably from about 0.25mm to about 0.5mm.

The skin-contactable element is preferably compressible, but not overly so. As such, in one embodiment, the skin-contactable element has a Compressibility of less than about 20%. In other embodiments, the Compressibility may be less than about 19%, or more preferably less than about 15%. Most preferably, the Compressibility may range from about 3% to about 13%.

The inventors have also noted that it is desirable for the skin-contactable element to have a thickness that is from about 0.1mm to about 20mm, preferably from about 0.5mm to about 5mm, more preferably from about 1mm to about 5mm, and most preferably from about 1.5mm to about 4.5mm. Thickness may be determined as the "Initial Thickness" in the Compressibility and Displacement Test, below. Other suitable properties of the skin-contactable element are described in commonly assigned, published U.S published patent application US20070010828 "Material for Mechanical Skin Resurfacing Techniques, hereby incorporated by reference"

The skin-contactable element 9 may further include a coating formed about or across the fibers, and, in one embodiment, formed atop the fibers 45 and atop the abrasive system as well. The coating may be least partially water-soluble such that in use, one or more ingredients within the coating dissolve in use and are transferred to the skin.19. In one embodiment of the invention, the coating is substantially free of abrasive, such as abrasive particles that could be transferred to and embed in the skin. In one embodiment of the invention, the coating is substantially free of water (i.e., includes less than about 2%, such as less than about 0.5% of water).

The coating may be formulated for one or more of various functions. For example, the coating may provide lubrication, emolliency, or and/or moisturization; mild foaming; a vehicle to deliver various benefit agents (e.g., benefit agents, drugs, and the like); or combinations thereof.

Furthermore, the coating 53 may include one or more benefit agents such as anti-acne agents, anti-wrinkle agents, anti-microbial agents, anti-fungal agents, antiinflammatory agents, topical anesthetic agents, artificial tanning agents, accelerator agents, anti-viral agents, enzyme agents, sunscreen agents, anti-oxidant agents, skin exfoliating agents, depilatory agents, and the like. Other suitable benefit agents are described in co-pending published patent application US2005-0148907, filed December 24, 2003, entitled "TREATMENT OF SKIN USING A BENEFIT AGENT AND AN APPARATUS," and co-pending patent application serial number 11/023655, filed December 28, 2004, entitled "SKIN TREATMENT ARTICLES AND METHODS, both cited previously.

### METHODS OF USE

System 1 of the present invention may be used to treat the skin, such as abrasive treatment, cleansing, or other skin treatments (e.g., acne, anti-aging, firmness, tone and texture, hair removal, body shaping/cellulite removal, and the like).

In one embodiment of the invention, the skin-contactable element is temporarily attached to the hand-held motorized apparatus. In another embodiment the skin-contactable element provides the abrasiveness and no additional abrasive is required. In another embodiment a composition (e.g., a suspension, emulsion, etc.) that includes abrasive particles is added to the skin-contactable element or to the skin to be treated.

A user grasping the apparatus (e.g., via precision grip described with reference to Figure 3) and turns the switch on the apparatus and imparts sufficient energy to the device to cause the apparatus to provide periodic motion such as vibration. For example, activation of the apparatus may cause the motor shaft to rotate and the eccentric weight coupled thereto to move in an eccentric orbit about the shaft. The vibration energy thus created from the rotating eccentric weight is transmitted to the abrasive. The vibrating abrasive contacts the skin providing benefits thereto..

The skin-contactable surface is moved across the face or other expanse of skin to be treated. For example, skin-contacting surface 11 (e.g., a substantially planar skin-contacting surface) is placed into contact with the skin to be treated. The skin-contactable surface provides, for example, increased cell proliferation by abrasively treating the skin. The skin-contactable element may have incorporated therewith a formulation to provide emolliency, foam, or delivery of benefit agents to the skin. When the user is finished, the skin-contactable surface may be removed and later replaced with a fresh one to provide a hygienic surface. The user may optionally rinse the treated skin after completion.

The inventors have discovered that by employing the methods of the present invention, various benefits such as cell proliferation, microdermabrasion efficacy, cleansing, and the like may be enjoyed with the added convenience of using a device that, in certain embodiments, can be made small, discrete, light, and cost-effective.

### TEST METHODS

### ABRASIVENESS TEST

"Durable Abrasiveness" is determined using the test method described below. The "Plain Abrasiveness" of a material is determined similarly to the Durable Abrasiveness, but the initial washing step is eliminated.

Five (5) samples of each of the skin-contactable elements to be tested are cut to a circular shape having a diameter of about 41 mm. Samples are individually rinsed with water in order to remove any materials such as foaming agents, oils, and emulsifiers that are readily separated from the article via contact with water. The cut samples are immersed in a bath of containing a mass of deionized water (temperature of about 35 °C) that has sufficient mass of water to be at least about 20 times the mass of the article. The article is allowed to remain in the bath for two minutes and is removed, allowed to drip for 10 seconds and then placed in another similar (fresh) water bath for two minutes, and again allowed to drip for 5 minutes. The sample is removed and allowed to dry at ambient temperature (at about 50-60% relative humidity) for a period from about 16 hours to about 72 hours. Again, this washing step is eliminated when measuring the Plain Abrasiveness.

After the sample is rinsed and dried as above, it is tested for abrasion using an abrasion testing device according to a modified version of ASTM test method D 3886-99. A suitable device is the CSI Universal Wear Tester, Model CS-226-605, available from Custom Scientific Instruments of Whippany, NJ. A sample of co-extruded spunbond/pigmented polyethylene film laminate (Clopay M18-1057, a 26 gsm laminate having (1) a 15 gsm (nominal) spunbonded polypropylene nonwoven web layer that is coextruded with (2) a 20 gsm (nominal) polyethylene film having a thickness of about 0.7 mils (0.007 inches), in which the polyethylene film surface of the laminate is corona treated, and the laminate has a target bond strength of 150 grams per inch, commercially available from Clopay Plastic Products of Mason, OH) is placed over the stage with the film oriented up, and the laminate is secured firmly against the stage with an o-ring, as supplied with the wear tester. The sample to be tested is secured on the arm above the stage such that it aligns directly on top of the stage. The sample is secured (preferably by tough double-sided tape - e.g., PERMACEL tape available from Permacel Company of East Brunswick, NJ in a manner such that the sample does not move when the tester is in operation. A 10 1b weight is loaded on the stage and the tester motor is powered. The stage simultaneously rotates and translates at a rate of about 130 cycles per minute. The number of cycles to failure is recorded as the first cycle in which the film is torn (for a pigmented, e.g., blue, film, the white spunbond readily shows through, marking the endpoint of the test. The process is repeated for the remaining samples. The average number of cycles to failure is recorded and a value for "Durable Abrasiveness" (for the washed samples) or for "Plan Abrasiveness" (for the unwashed samples) is calculated as 2000 divided by the average cycles to failure.

A standard sample, SCOTCH-BRITE Pad ("Heavy Duty Commercial Scoring Pad," # 86) is desirably run as a standard with each data set. The SCOTCH-BRITE Pad, # 86 should yield a Durable Abrasiveness value of approximately 33 +/- 4. If the operator determines a Durable Abrasiveness that falls outside this range, this signifies slight operator error, and the operator should adjust any subsequent determinations for Durable Abrasiveness by a factor that corrects for this operator error. That factor is (V/33), where V is the value determined by the operator for SCOTCH-BRITE Pad, # 86. If SCOTCH-BRITE Pad, # 86 is not available, then, as a substitute, SCOTCH-BRITE Pad ("General Purpose Commercial Scoring Pad," #96") can be run as a standard, with the expected value of Durable Abrasiveness as 14 +/- 2 and a correction factor of (V/14) if this alternative standard does not fall within the prescribed range.

The Abrasiveness value for the five samples is averaged and reported as the Durable Abrasiveness or Plain Abrasiveness value for the particular skin-contactable element.

### COMPRESSIBILITY AND DISPLACEMENT TEST

"Displacement" is determined using the following test method: for each article to be tested, five samples are cut to a size of about 41mm diameter. One at a time, a sample is placed on a thickness gauge such as the Ames Logic Plus (model LG3601-1-04) available from BC Ames of Waltham, MA, and the sample is centered under the 55mm foot. A 0.5 oz weight is placed on the shaft and the foot is gently lowered onto the sample. The "Initial Thickness" reading is taken after the gauge is allowed to stabilize for 10 seconds. Next, the foot is lifted, the 0.5 oz. Weight is replaced with an 8 oz weight. After the gauge is allowed to stabilize for 10 seconds, the "Thickness Under Load" is recorded. The process is repeated for 10 samples. For each sample the difference between Initial Thickness and Thickness Under Load is calculated and recorded. The result for the 10 samples is averaged and recorded as the Displacement for the particular skin-contactable element.

"Compressibility" is calculated as the Displacement of a sample divided by its Initial Thickness and expressed as a percent. The result for the 10 samples is averaged and recorded as the Compressibility for the particular skin-contactable element.

### EXAMPLES

The following examples relate to skin-contactable elements of the present invention. Other embodiments of the invention can be prepared in an analogous manner by a person of ordinary skill in the art.

### Examples 1-4

A clinical assessment was performed in which a make-up foundation, "REVLON Colorstay Cappuccino 410 Make-Up with Softflex" (commercially available from Revlon Consumer Products Corporation of New York, NY) was applied independently to 10 female subjects (ages 18-45, Fitzpatrick skin types I, II, and III) by a clinician. Five tests sites were chosen on each subject's volar forearm (2 or 3 sites per arm). About 70 microliters of make-up was applied to each site using a 5 cm by 5 cm template to restrict the application of the make-up to the particular site. The make-up was spread evenly across each site with a gloved finger. Both arms were then positioned under a hair drier for 5 minutes on high setting to uniformly dry the make-up treated sites.

Photographic images were taken on the make-up treated sites under a fixed luminescence level selected by the clinician to provide a high level of image contrast. One site for each subject was selected as a tape-stripping (control) site. This site was sprayed with a light film of water positioned 6 inches from the site. The site was then blotted (not rubbed) with a small KIM-WIPE (Kimberly Clark Neenah, Wisconsin) towelette. A D-SQUAM tape (commercially available from CuDerm Corporation of Dallas, Texas) was applied in the center of the test site. Using the tape applicator, the tape was pressed onto the skin to ensure even adhesion. The tape was left on for 50 seconds and then removed. A digital image was then taken of the site. The process was repeated such that 10 tape strips were performed. Additional tape strips were performed, but another image was not taken until the 15 tape strip removal and then again at the 20^{th} tape strip removal.

In order to determine the dependence of make-up removal as a function of number of tape strips, a pixel counting image analysis was performed. Image selection was performed using Adobe Photoshop software available from Adobe System Inc. of San Jose, California. The approximate center of each site was identified and a circular region having a diameter of 240 pixels was selected, inverted, cropped and back filled with green (R=0, G=255, B=0). The number of pixels below a pre-selected luminescence level was recorded as the "number of dark pixels." The cutoff value to categorize a pixel as "dark" or "not dark" was independently and arbitrarily selected for each subject in the study to correlate to the clinician's visual inspection. Once the cutoff was determined, the same cutoff was used for all skin treatments for that particular subject. The determination of number of dark pixels was repeated for each image taken. On the remaining 4 sites per subject, a cleansing treatment protocol was performed.

The four treatments are summarized in Table 1 below, and details of the particular treatments are provided

**Table 1**

| TREATMENT IDENTIFIER | TREATMENT SUMMARY |
|---|---|
| Inventive Example, Ex. 1 | Non-woven skin-contactable element with coating; used with inventive shaped, skin treatment apparatus |
| Comparative Example, C1 | Cetaphil gentle skin cleanser, no apparatus (available from Galderma Labs, Fort Worth, Texas) |
| Comparative Example, C2 | DOVE Skin VITALIZER with Gentle Exfoliating Daily Facial Cleansing Pillows (Commercially available from Unilever of Englewood Cliffs, NJ) |
| Comparative Example, C3 | Pond's Clean Sweeo Cleansing and Make-Up Removal Towelettes (Commercially available from Unilever of Englewood Cliffs, NJ) |

### Inventive Example, E1

The site to be treated was rinsed with one spray of water positioned six inches from the site. A skin-contactable element (pad) was secured to a carrier having a loop (e.g., VELCRO) attachment bonded thereto. The carrier was snapped onto an apparatus having a vibrating motor. Three milliliters of water was deposited on the center of the pad. The device was energized and rotated about the site for 10 circular motions at a rotation rate of about 1 rotation per second. The site was then rinsed with 7 sprays from the designated spray bottle positioned 6 inches from the site. The site was allowed to air dry.

The motorized apparatus used was one similar to that described with respect to Figures 1-7. The motor employed in the apparatus had an eccentric mass of about 1.6 grams. The apparatus had a maximum linear dimension of less than about 9 cm, a dimension 101 of about 6.7cm, a projected area of about 20cm², an area of attachment surface about 11.3 cm², and surrounded a volume of about 83 cm³,

The skin-contactable element was a freestanding fibrous, non-woven material (a needlepunched blend of 50% LYOCEL (1.4 denier, 2.0 inch length) and 50% polyester (a mixture of 3 denier, 3 inch length and 1.5 denier, 3 inch length), having a basis weight of about 300 gsm, available from Precision Custom Coating of Totowa, NJ, USA). The non-woven was cut into a circular pad having a diameter of 4.1 cm. A cleansing composition was then applied to the top surface of the composite non-woven. The cleansing composition included the following ingredients to form a base cleanser:

**Table 2**

| Trade Name | Chemical Name | % (w/w) |
|---|---|---|
| Texapon NC70 | Sodium Laureth Sulfate | 29.39 |
| Tegobetaine F-50 | Cocamidopropyl Betaine | 18.16 |
| Plantaren 2000 N | Decyl Glucoside | 5.04 |
| Carbowax PEG 400 | PEG-8 | 12.04 |
| Glucquat 125 | Lauryl Methyl Gluceth-10 Gluceth-10 Hydroxypropylammonium chloride Chloride | 1.0000 |
| Phenoxetol | Phenoxyethanol | 0.58 |
| Methyl Paraben | Methyl Paraben | 0.16 |
| Propyl Paraben | Propyl Paraben | 0.10 |
| Citric Acid anhydrous | Citric Acid | 0.25 |
| Emery 917 | Glycerin | 33.28 |

To form the coating, the glycerin was added to a beaker. The butylparaben, methylparaben, and propylparaben were added thereto and slowly mixed until the parabens dissolved. The PEG-8 and glucquat were then added to the beaker and mixed. The Cocamidopropyl Betaine, Sodium Laureth Sulfate, Decyl Glucoside, and Phenoxyethanol were then added and mixed. The citric acid was then added and the ingredients mixed until the citric acid was completely dissolved. The pH was adjusted to between 6.4 and 7.2 with citric acid and/or sodium hydroxide.

The base cleanser above (99.25%) was heated and mixed with sufficient salicylic acid and menthol to yield 0.5% salicylic acid and 0.25% menthol and 99.25% base cleanser. The pH was adjusted to between 6.4 and 7.2 with citric acid and/or sodium hydroxide, forming an unfragranced cleanser. Unfragranced cleanser (99.14%) was mixed with 0.86% Givaudan About Face fragrance to form the final cleansing composition, which was coated on the pad.

### Comparative Example, C1

The site was rinsed as described above for Inventive Example 1. 100 microliters of CETAPHIL Gentle Skin Cleanser (available from Galderma Labs, Fort Worth, Texas) as applied to the test site and massaged into the test site using a gloved finger for 10 circular motions (at approximately 1 rotation per second). The site was rinsed and dried as described above for Inventive Example 1.

### Comparative Example, C2

The site was rinsed as described above for Inventive Example 1. A Gentle Exfoliating Daily Facial Cleansing Pillow was affixed to loop attachment on DOVE Skin VITALIZER (Commercially available from Unilever of Englewood Cliffs, NJ) with abrasive side facing the area to be treated. The device was powered and placed into contact with the test site. The device was rotated for 10 circular motions at approximately 1 rotation per second. The site was rinsed and dried as described above for Inventive Example 1.

### Comparative Example, C3

The site was rinsed as described above for Inventive Example 1. A Pond's Clean Sweep Cleansing and Make-Up Removal Towelette (commercially available from Unilever of Englewood Cliffs, NJ) was applied to the test site to the area to be treated. The cloth was rotated for 10 circular motions at approximately 1 rotation per second. The site was rinsed and dried as described above for Inventive Example 1.

After all sites were treated with the various make-up removing treatments, all of the sites were dried with a hair drier for one minute on high setting. Digital images were captured for each site.

The percent makeup removal, shown in Chart 1, was determined for the various skin treatments by determining the fraction of the original dark pixels were no longer dark after the treatment (due to the treatment having removed the dark make-up). The inventive skin treatment and apparatus of the present invention had superior make-up removal compared with the comparative examples. Specifically, treatment according to the inventive example exhibited 72% removal, as compared to the comparative examples which varied between about 56.7% removal (DOVE) to 46.9% (PONDS) to as low as 0.1% removal (CETAPHIL).

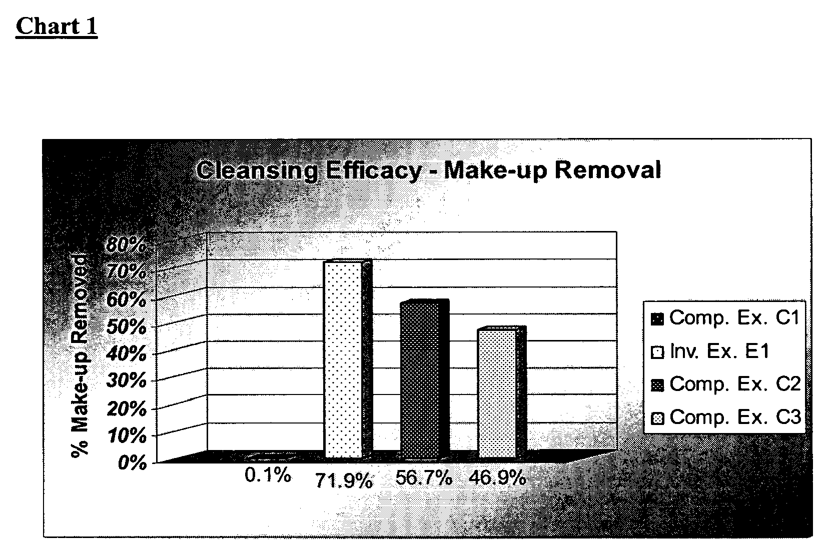

Chart 2 shows a plot of number of dark pixels (make up remaining on the skin) as a function of number of tape strips (downward sloping curve) and as a function of the removal treatment. Treatment according to the inventive example, Ex. 1 exhibited removal equivalent to 18 tape strips, as compared to the comparative examples that varied between about 9 tape strips (DOVE) to 8 tape strips (PONDS) to as low as less than 1 tape strip (CETAPHIL).

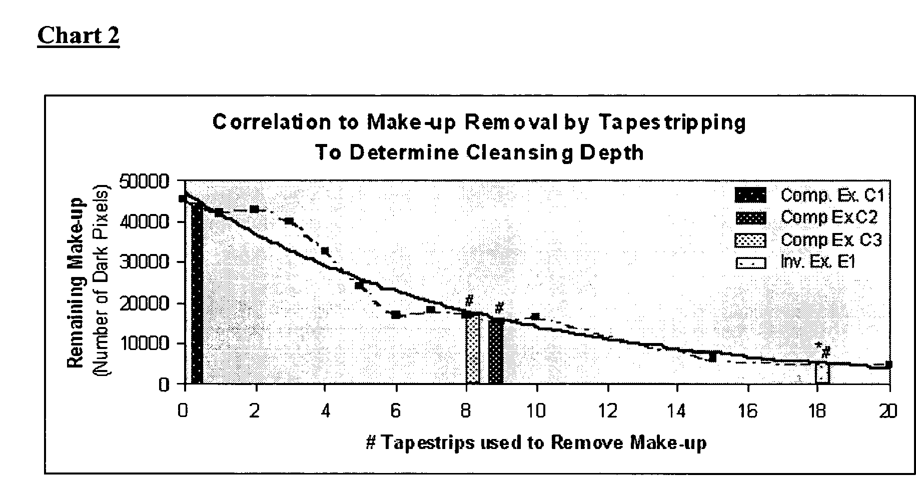

## Claims

1. A handheld, motorized apparatus arranged and configured to impart motion to skin placed in contact therewith, wherein the motorized apparatus comprises:
a body comprising:
an attachment surface suitable for coupling a skin-contactable element thereto;
a motor within said body; and
a means for transferring mechanical energy from the motor to the skin-contactable element in order to impart motion to the skin contactable surface; wherein said apparatus has a maximum linear dimension of less than about 13 cm.

2. The handheld, motorized apparatus of claim 1, wherein said device has a maximum linear dimension of less than about 11 cm, preferably of less than about 9 cm.

3. The handheld, motorized apparatus of claim 1 or claim 2, wherein said attachement surface has an area of at least about 5 cm², preferably at least about 10cm².

4. The handheld, motorized apparatus of any preceding claim, wherein said apparatus defines a volume of less than about 130 cm³, preferably less than about 100cm³.

5. The handheld, motorized apparatus of any preceding claim, wherein said motor comprises a rotatable shaft and weight coupled to the shaft, such that said weight is capable of eccentric motion about said shaft, and wherein said weight has a mass less than about 4 grams.

6. The handheld, motorized apparatus of claim 5, wherein said weight has a mass less than about 3 grams, preferably less than about 2 grams.

7. The handheld, motorized apparatus of claim 5, wherein said weight has a mass of from about 1.6 to about 4 grams.

8. The handheld, motorized apparatus of any preceding claim, wherein said apparatus has a mass of less than about 125 grams, preferably less than about 100 grams.

9. A handheld, motorized apparatus according to any preceding claim wherein the body additionally comprises:
a first gripping surface; and
a second gripping surface opposed to said first gripping surface, wherein said first gripping surface and said second gripping surface are sized and configured to be held between distal portions of a thumb and fingers of a user's hand at a distance from a corresponding palm of said user.

10. The handheld, motorized apparatus of claim 9, wherein said first gripping surface and said second gripping surface are convex.

11. The handheld, motorized apparatus of claim 9 or claim 10, wherein said first gripping surface and said second gripping surface have a minimum separation of from about 10 cm to about 50 cm, preferably from about 15 cm to about 30 cm.

12. The handheld, motorized apparatus of any preceding claim, wherein said attachement surface is substantially planar.

13. A handheld, motorized apparatus arranged and configured to impart motion to skin placed in contact therewith, wherein the motorized apparatus comprises:
a substantially planar attachment surface suitable for coupling a skin-contactable element thereto;
a motor within said body; and
a means for transferring mechanical energy from the motor to the skin-contactable element in order to impart motion to the skin contacable surface; wherein said apparatus has projected area less than about 100 cm², wherein said projected area is the area projected in a direction normal to said substantially planar surface, and wherein said apparatus has a dimension that is measured from said substantially planar attachment surface along said direction to a point on said device that is most remote from said substantially planar attachment surface, and wherein said dimension is less than about 11 cm.

14. A method for treating the skin, comprising:
a) energizing the motor of the apparatus of any of Claims 1 to 13; and
b) contacting said skin with said skin-contactable element for sufficient time to treat the skin.

15. A kit, comprising:
a) the apparatus of any of Claims 1 to 13; and
b) a skin-treatment pad.
